# EUROPEAN PATENT APPLICATION

(11) **EP 4 252 861 A1**
(43) Date of publication of application: **04.10.2023**
(21) Application number: 21897904.5
(22) Date of filing: 22.11.2021
(51) Int. Cl.: A61Q 11/00, A61K 8/21, A61K 8/24, A61K 8/64, A61K 8/98

(54) **TOOTH STRENGTHENING METHOD AND TOOTH WHITENING METHOD**

(30) Priority: 24.11.2020 JP 2020194765
(71) Applicant: Nishio Co., Ltd., Chiba-shi, Chiba 260-0012 (JP)
(72) Inventor: NISHIO, Hidetoshi, Chiba-shi, Chiba 260-0012 (JP)
(74) Representative: Zaboliene, Reda
(86) International application number: PCT/JP2021/042794
(87) International publication number: WO 2022/113931

(57) **Abstract**

It is an object of the present invention to provide a tooth substance strengthening method capable of strengthening tooth substance easily and effectively. It is another object of the present invention to provide a tooth whitening method capable of whitening teeth while at the same time strengthening tooth substance by means of the tooth substance strengthening method. The tooth substance strengthening method according to the present invention is characterized in that a solvent 14 obtained by mixing calcium phosphate as a main component with the subject's own saliva is applied to the surface of the tooth 1 . Further, the method for whitening the tooth 1 according to the present invention includes: a first step of stripping off the pellicle 11 covering the tooth surface with a cleansing agent 12; a second step of applying a whitening agent 13 to the surface of the tooth 1; a third step of applying a solvent 14 obtained by mixing an agent containing calcium phosphate as a main component with the subject's own saliva to the surface of the tooth 1; and a fourth step of applying fluorine 16 to the surface of the tooth 1.

## Description

### Technical Field

The present invention relates to a tooth substance strengthening method and a tooth whitening method including the tooth substance strengthening method.

### Background Technology

One method for dental caries prevention and their treatments is to strengthen the tooth structure, and as one of such methods it has been commonly practiced to apply fluorine to tooth surface.

On the other hand, tooth enamel is the hardest tissue in the human body, and approximately 96% of it is made up of crystals of hydroxyapatite (Ca₁₀(PO₄)6(OH)₂) which is one kind of calcium phosphate. Although this hydroxyapatite is composed of calcium, phosphoric acid, and hydroxyl groups at the molecular level, it has a property of being weak against acids.

Further, plaque (dental plaque) adheres to the surface of teeth, and the activity of bacteria in the plaque generates acid. When hydroxyapatite, which constitutes tooth enamel, comes into contact with acid, calcium and phosphoric acid will dissolve (demineralize) from crystal components of hydroxyapatite, forming a cause of tooth decay.

Moreover, calcium that dissolves from the crystalline component of hydroxyapatite will dissolve in saliva. However, once the acid produced by plaque disappears, the calcium dissolved in saliva will be re-incorporated into the enamel and recrystallize to the original hydroxyapatite (remineralization). At this time, if a small amount of fluoride is present in saliva, this will promote remineralization and prevent tooth decay. Namely, hydroxyapatite becomes fluoroapatite (Ca₁₁₀ (PO₄) ₆F₂ ) by virtue of fluorine, and enamel is strengthened by fluoroapatite (which has a high acid resistance), and becomes less prone to become caries.

On the other hand, in the field of dentistry, in addition to the treatment of tooth decay, there is a demand to make the tooth surface look whiter (whitening) from an aesthetic point of view. Possible causes of yellowing of teeth include staining due to the adhesion of tobacco tar and the like, a decrease in detergency in the mouth due to a decrease in saliva which is caused due to mouth drying, and also due to thinning of teeth.

A relationship between tooth thinning and tooth yellowing can be explained as follows. Namely, when the translucent enamel of the tooth becomes thin due to excessive brushing or the like, yellowish portions called dentin inside the tooth will become transparent and looks yellowish.

On the other hand, various proposals have been made so far regarding tooth whitening methods (see, for example, Patent Documents 1 - 3).

### Prior Art Documents

### Patent Literatures

Patent Document 1: Japanese Patent Publication No. 2006-508892
Patent Document 2: JP-A-2009-062397
Patent Document 3: JP-A-2009-268689

### Summary of the Invention

### Problems to be Solved by the Invention

However, the tooth whitening methods proposed in Patent Documents 1-3 fail to strengthen the tooth substance, and cannot solve the problem that the teeth look yellowed due to teeth thinning which is caused due to excessive brushing.

On the other hand, the entire tooth surface is covered with a thin organic film called a pellicle. Even if fluorine is applied to the tooth surface covered with this pellicle, the effect of strengthening the tooth substance by fluorine will be reduced by half.

The present invention has been accomplished in view of the above problems, and it is an object of the present invention to provide a method for strengthening tooth substance so as to strengthen tooth structure easily and effectively, and a tooth whitening method capable of whitening teeth while strengthening tooth structure using a tooth substance strengthening method.

### Means to Solve Problems

In order to achieve the above object, the method for strengthening tooth substance according to the present invention comprises: applying a solvent (14) to the surface of the tooth (1), said solvent (14) being obtained by mixing an agent containing calcium phosphate as a main component with a subject's saliva.

According to the present invention, statherin contained in saliva and calcium contained in calcium phosphate react with each other to promote remineralization of tooth enamel, forming a strong calcified film on the tooth surface and thus effectively strengthening the tooth substance. In this way, it is possible to easily obtain the above-mentioned effect by simply applying the solvent to the tooth surface.

Here, in the above method for strengthening tooth substance, fluorine (16) may also be applied to the surface of the tooth (1) after the solvent (14) is applied to the surface of the tooth (1).

According to the above method, the crystals of the strongly calcified film formed on the tooth surface in the previous step are densified by the fluorine, and the tooth surface is effectively protected by the densified and hardened strongly calcified film, thereby making it possible to more effectively strengthen the tooth substance.

Further, it is also possible to use a cleansing agent (12) to strip off the pellicle (11) covering the tooth surface, before applying the fluorine (16) to the surface of the tooth (1).

According to the above method, the effect of strengthening tooth substance by fluorine can be enhanced. On the other hand, if the pellicle remains on the tooth surface, the effect of strengthening the tooth substance with fluorine will be reduced by half.

The tooth whitening method according to the present invention comprises: a first step of removing the pellicle (11) covering the tooth surface with a cleansing agent (12); a second step of applying a whitening agent (13) to the surface of the tooth (1); a third step of applying a solvent (14) to the surface of the tooth (1), said solvent being obtained by mixing an agent containing calcium phosphate as a main component with the subject's own saliva; and a fourth step of applying fluorine (16) to the tooth (1).

According to the present invention, the pellicle covering the entire tooth surface is stripped off in the first step, and in the subsequent second step, a whitening agent is applied to the tooth surface from which the pellicle has been removed, thereby bleaching the teeth (whitening teeth). Then, in the next third step, a solvent obtained by mixing the subject's own saliva with an agent containing calcium phosphate as a main component is applied to the surface of teeth, so that the statherin contained in the saliva and the calcium contained in the calcium phosphate are reacted with each other, thereby strengthening the tooth substance. In the subsequent fourth step, fluorine is applied to the tooth surface from which the pellicle has been removed, thereby densifying the enamel crystals on the tooth surface, thus further strengthening the tooth substance. As a result, teeth can be whitened while at the same time strengthening the tooth structure.

### Effect of the Invention

According to the present invention, it is possible to provide a tooth substance strengthening method capable of easily and effectively strengthening tooth substance, and a tooth whitening method capable of whitening teeth while at the same time strengthening tooth substance using this tooth substance strengthening method.

### Brief Description of the Drawings

Figs. 1A - 1C are explanatory views showing a tooth substance strengthening method of the present invention, in accordance with the order of steps included therein.
Figs. 2A - 2D are explanatory views showing a tooth whitening method of the present invention, in accordance with the order of steps included therein.

### Embodiments for Carrying out the Invention

Embodiments of the present invention will be described below.

### [Tooth Substance Strengthening Method]

First, the tooth substance strengthening method according to the present invention will be described below.

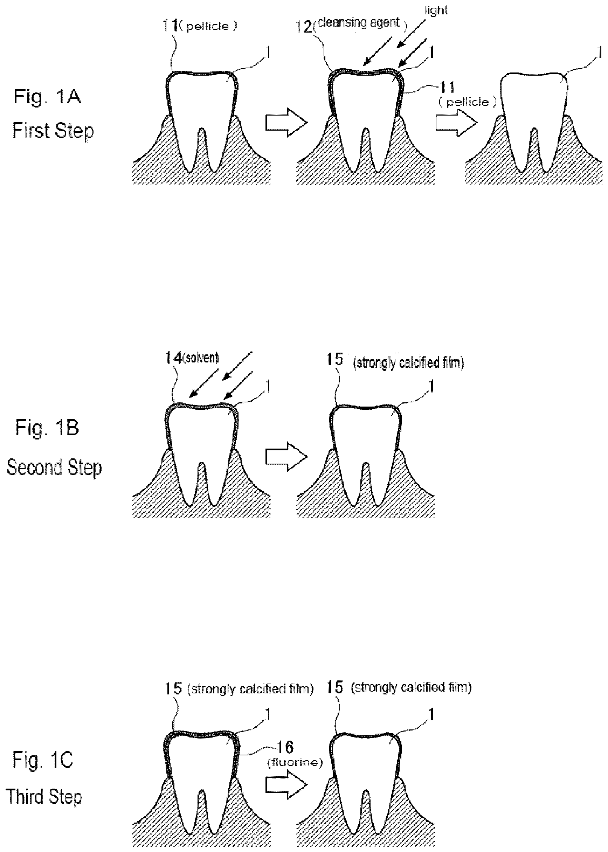

Figs. 1A - 1C are explanatory views showing the tooth substance strengthening method of the present invention, according to the steps included therein. In the present embodiment, the following three steps are successively carried out.

### Fig. 1A - first step:

In the first step, the thin pellicle (organic film) 11 covering the entire surface of the tooth 1 is stripped off. Specifically, as shown in the figure, an acidic cleansing agent 12 is applied to the surface of the tooth 1. Then, a light (laser light or plasma light) is applied to the tooth surface. Subsequently, the peeled pellicle 11 and the excess cleansing agent 12 are removed with polyphosphoric acid liquid. In this way, the pellicle 11 covering the entire tooth surface can be removed.

### Figure 1B - second step:

In the second step, the surface of the tooth 1 from which the pellicle 11 has been removed in the first step is coated with a solvent 14, which is a mixture of an agent containing calcium phosphate as a main component and the subject's own saliva, followed by being irradiated with a light (laser light or plasma light). Then, the excess solvent 14 is wiped off with a polyphosphoric acid liquid. In this way, the surface of the tooth 1 can be coated with a strongly calcified film 15.

Here, the calcium phosphate contained in the agent (liquid agent or powder agent) is a chemical combination of phosphoric acid (H₃PO₄) and calcium (Ca). When the subject's own saliva is mixed with the agent containing this calcium phosphate, the statherin and calcium contained in the saliva combine together to form a strongly calcified film 15 on the surface of the tooth 1, thereby strengthening the tooth substance by virtue of the strongly calcified film 15.

### Fig. 1C-third step:

In the third step, fluorine 16 is applied to the surface of the tooth 1 coated with the strongly calcified film 15 in the second step, and then excess fluorine 16 is wiped off. In this way, the crystals of the strongly calcified film 15 formed on the surface of the tooth 1 are densified to further strengthen the tooth substance.

Specifically, the presence of a small amount of fluoride in saliva can promote remineralization and thus prevent tooth decay. Namely, hydroxyapatite (Ca₁₀(PO₄)₆(OH)₂) constituting hard enamel covering the surface of the tooth 1 becomes fluoroapatite (Ca₁₁₀(PO₄)₆F₂) by virtue of fluorine 16, while fluoroapatite having a high acid resistance, can strengthen enamel and prevent tooth decay.

According to the tooth substance strengthening method of the present embodiment, the tooth substance can be effectively strengthened and the occurrence of caries can be prevented, simply by successively performing the above first to third steps.

On the other hand, it should be noted that the first step (stripping off the pellicle 11) and the third step (applying the fluorine 16) in the present embodiment are not absolutely essential, and may be carried out only when necessary.

### [Teeth whitening method]

Next, the tooth whitening method according to the present invention will be described below.

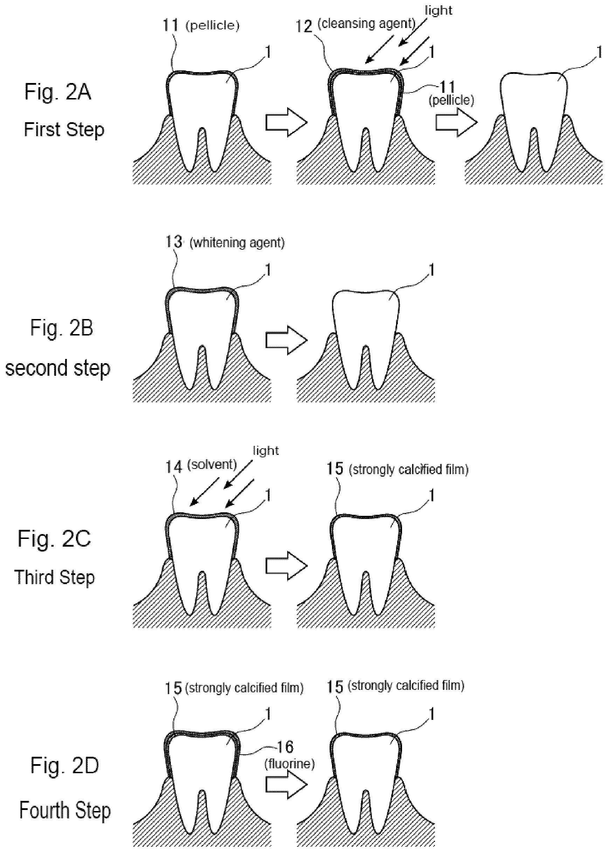

Figs 2A - 2D are explanatory views showing the tooth whitening method of the present invention, in accordance with the order of steps included therein. In the tooth whitening method according to the present embodiment, teeth are whitened (bleached) through the following four steps.

Here, the first, third and fourth steps shown below are the same as the first, second and third steps in Fig. 1 for explaining the tooth substance strengthening method. The tooth whitening method according to the present embodiment is formed by incorporating a tooth whitening step as a second step between the first step (stripping off the pellicle 11) and the second step (forming the strongly calcified film 15 by applying the solvent 14) shown in Fig. 1. Namely, in the tooth whitening method according to the present embodiment, the following first to fourth steps are successively carried out.

### Fig. 2A - first step:

Since this first step is the same as the first step (Fig. 1A) in the tooth substance strengthening method, a re-explanation thereof is omitted here.

### Figure 2B - second step:

In the second step, a whitening agent 13 made by adding sodium polyphosphate to hydrogen peroxide is applied to the surface of the tooth 1 from which the pellicle 11 has been stripped of in the first step. In this way, the tooth 1 can be bleached and whitened by the bleaching function of the whitening agent 13, while the excess whitening agent 13 can be wiped off the surface of the tooth 1. On the other hand, for use as the whitening agent 13, it is also possible to use any other agent.

### Fig. 2C - third step:

In the third step, the surface of the tooth 1 that has been whitened in the second step is coated with a solvent 14, which is a mixture of an agent mainly composed of calcium phosphate and the subject's own saliva, followed by applying a light (laser light or plasma) to the tooth surface. Then, the excess solvent 14 is wiped off with a polyphosphoric acid liquid. The surface of the tooth 1 is then coated with a strongly calcified film 15. Since the third step is the same as the second step shown in Fig. 1B described in the tooth substance strengthening method, a further detailed description thereof is omitted here.

### Fig. 2D - fourth step:

In the fourth step, fluorine 16 is applied to the surface of the tooth 1 coated with the strongly calcified film 15 in the third step, and then excess fluorine 16 is wiped off. Since this step is the same as the third step in Fig. 1C described in the tooth substance strengthening process, the re-description thereof will be omitted here.

According to the method of the present invention, upon successively carrying out the above first to fourth steps, it is possible to obtain an effect that the tooth 1 can be whitened while at the same time strengthening the tooth substance.

The present invention is not limited to the embodiments described above, and various modifications are possible within the scope of the technical ideas described in the claims, specification and drawings.

## Claims

1. A method for strengthening tooth substance, **characterized by** applying a solvent to the surface of a subject's teeth, said solvent being a mixture of calcium phosphate as a main component and the subject's own saliva.

2. The method according to claim 1, wherein fluorine is applied to the tooth surface after the solvent is applied to the tooth surface.

3. The method according to claim 2, wherein the pellicle covering the tooth surface is stripped off by using a cleansing agent before the fluorine is applied to the tooth surface.

4. A method for whitening teeth, comprising:
a first step which removes pellicle covering tooth surface by using a cleansing agent;
a second step which applies a whitening agent to the tooth surface;
a third step which applies a solvent to the surface of a subject's teeth, said solvent being a mixture of calcium phosphate as a main component and the subject's own saliva; and
a fourth step which applies fluorine to the tooth surface.
